# EUROPEAN PATENT APPLICATION

(11) **EP 4 285 833 A1**
(43) Date of publication of application: **06.12.2023**
(21) Application number: 22187378.9
(22) Date of filing: 28.07.2022
(51) Int. Cl.: A61B 8/00, A61B 5/02

(54) **ULTRASOUND SYSTEMS AND PATIENT MONITORING SYSTEMS**

(30) Priority: 02.06.2022 US 202263348189 P
(71) Applicant: Koninklijke Philips N.V., 5656 AG Eindhoven (NL)
(72) Inventor: SUTTON, Jonathan Thomas, Eindhoven (NL); POLAND, McKee, Eindhoven (NL); GREINER, Harald, 5656AG Eindhoven (NL); BRADLEY, Kevin, 5656AG Eindhoven (NL)
(74) Representative: Philips Intellectual Property & Standards

(57) **Abstract**

According to an aspect, there is provided a method of operating an ultrasound system (2) that is for acquiring ultrasound images of a first subject. The method comprises: performing (101) a time synchronization operation with an Internet-based time server (20) to update an internal clock of the ultrasound system (2); identifying (103) a patient monitoring system (4; 6) that is measuring one or more physiological characteristics of the first subject; establishing (105) a communication link with the identified patient monitoring system (4; 6); receiving (205) a series of measurements of the one or more physiological characteristics of the first subject from the patient monitoring system (4; 6), wherein the received series of measurements comprise respective timestamps indicating respective times of acquisition of the measurements by the patient monitoring system (4; 6); acquiring (107) a series of ultrasound images of the first subject, wherein the ultrasound images comprise respective timestamps, determined according to the internal clock, indicating respective times of acquisition of the ultrasound images; and synchronizing (109) the received series of measurements with the acquired series of ultrasound images according to the respective timestamps.

## Description

### FIELD OF THE INVENTION

This disclosure relates to ultrasound systems for acquiring ultrasound images of a first subject, and to patient monitoring systems for obtaining measurements of one or more physiological characteristics of the first subject.

### BACKGROUND OF THE INVENTION

In the clinical intensive care and patient monitoring environment, invasive methods for monitoring central cardiac parameters have been falling out of favor recently due to high rates of morbidity. Ultrasound is a trusted modality - the gold standard for definitive diagnosis and volume-based measures - but suffers from high inter- and intra-operator variability, particularly amongst non-expert users. Further, ultrasound use in the critical care environment is often siloed, lacking integration with patient monitoring networks and databases that provide real-time and past vital signs. For ultrasound to grow as a definitive, non-invasive tool in the perioperative, critical care environment, better data integration for real time acquisition and retrospective analyses are needed.

The integration of ultrasound into the digital care continuum is happening on several levels. Ultrasound image acquisitions have historically been guided by measurements of other physiological characteristics or signals representing other physiological characteristics, such as electrocardiogram (ECG) signals. Ultrasound images are reviewed in the context of measurements of other physiological characteristics, such as respiration and heart rate. Data derived from ultrasound images can be used as input to models that provide decision support for care teams, alongside a myriad of other data sources and types.

### SUMMARY OF THE INVENTION

One problem that prohibits deep integration of ultrasound with patient monitoring networks is the lack of time synchronization. Ultrasound systems and patient monitors and their central station counterparts tend to maintain separate time clocks. Certain embodiments of the teaching provided in this disclosure enable ultrasound systems to use data from patient monitoring systems for ultrasound image (a) acquisition, (b) display, and/or (c) review by using low latency wireless connections and a network time protocol for synchronization.

Aspects of the present disclosure provide methods for providing physiological characteristic measurements from a patient monitoring system to an ultrasound system to enable ultrasound images and synchronized physiological characteristic measurements to be displayed and/or stored in a data file for the patient.

According to a first specific aspect, there is provided a method of operating an ultrasound system that is for acquiring ultrasound images of a first subject. The method comprises: performing a time synchronization operation with an Internet-based time server to update an internal clock of the ultrasound system; identifying a patient monitoring system that is measuring one or more physiological characteristics of the first subject; establishing a communication link with the identified patient monitoring system; receiving a series of measurements of the one or more physiological characteristics of the first subject from the patient monitoring system, wherein the received series of measurements comprise respective timestamps indicating respective times of acquisition of the measurements by the patient monitoring system; acquiring a series of ultrasound images of the first subject, wherein the ultrasound images comprise respective timestamps, determined according to the internal clock, indicating respective times of acquisition of the ultrasound images; and synchronizing the received series of measurements with the acquired series of ultrasound images according to the respective timestamps.

According to a second aspect, there is provided a method of operating a patient monitoring system that is for obtaining measurements of one or more physiological characteristics of a first subject. The method comprises: performing a time synchronization operation with an Internet-based time server to update an internal clock of the patient monitoring system; establishing a communication link with an ultrasound system that is to acquire ultrasound images of the first subject; obtaining a series of measurements of the one or more physiological characteristics of the first subject, wherein the measurements comprise respective timestamps indicating respective times of acquisition of the measurements by the patient monitoring system; and sending the series of timestamped measurements of the one or more physiological characteristics of the first subject to the ultrasound system.

According to a third aspect, there is provided a computer program product comprising a computer readable medium having computer readable code embodied therein, the computer readable code being configured such that, on execution by a suitable computer or processor, the computer or processor is caused to perform the method according to the first aspect, the second aspect, or any embodiments thereof.

According to a fourth aspect, there is provided an ultrasound system that is for acquiring ultrasound images of a first subject. The ultrasound system is configured to: perform a time synchronization operation with an Internet-based time server to update an internal clock of the ultrasound system; identify a patient monitoring system that is measuring one or more physiological characteristics of the first subject; establish a communication link with the identified patient monitoring system; receive a series of measurements of the one or more physiological characteristics of the first subject from the patient monitoring system, wherein the received series of measurements comprise respective timestamps indicating respective times of acquisition of the measurements by the patient monitoring system; acquire a series of ultrasound images of the first subject, wherein the ultrasound images comprise respective timestamps, determined according to the internal clock, indicating respective times of acquisition of the ultrasound images; and synchronize the received series of measurements with the acquired series of ultrasound images according to the respective timestamps.

According to a fifth aspect, there is provided a patient monitoring system that is for obtaining measurements of one or more physiological characteristics of a first subject. The patient monitoring system is configured to: perform a time synchronization operation with an Internet-based time server to update an internal clock of the patient monitoring system; establish a communication link with an ultrasound system that is to acquire ultrasound images of the first subject; obtain a series of measurements of the one or more physiological characteristics of the first subject, wherein the measurements comprise respective timestamps indicating respective times of acquisition of the measurements by the patient monitoring system; and send the series of timestamped measurements of the one or more physiological characteristics of the first subject to the ultrasound system.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 illustrates an exemplary system arrangement to enable physiological characteristic measurements to be transferred from a patient monitoring system and/or a patient data storage system to an ultrasound system;
Fig. 2 illustrates data exchange using a slow protocol and a fast protocol;
Fig. 3 is a signaling diagram illustrating the operations of an ultrasound system and a patient monitoring system according to various embodiments; and
Fig. 4 is a block diagram illustrating an apparatus according to various embodiments.

### DETAILED DESCRIPTION OF EMBODIMENTS

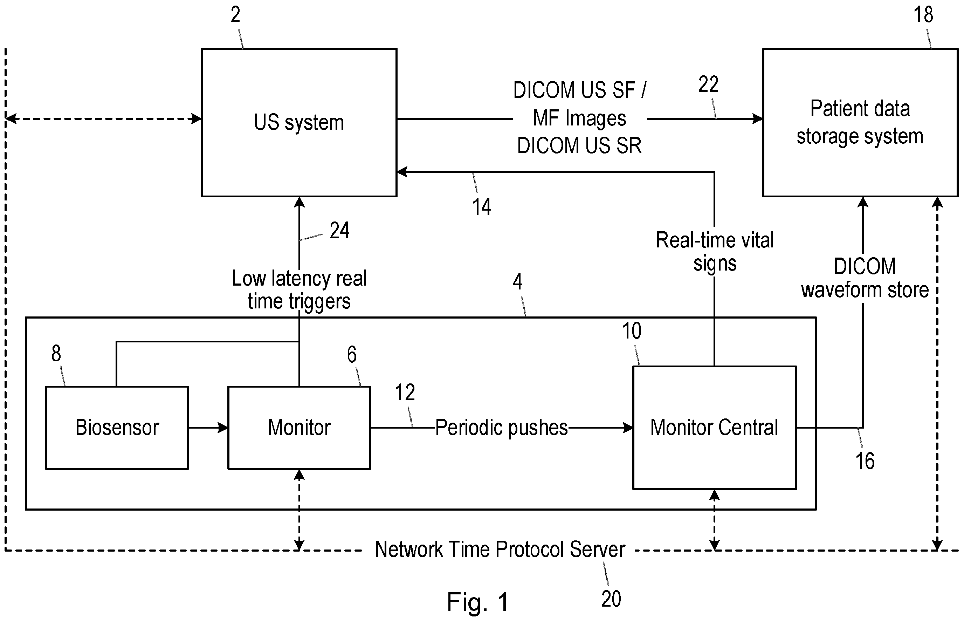

Fig. 1 illustrates an exemplary system arrangement to enable physiological characteristic measurements to be transferred from a patient monitoring system and/or a patient data storage system to an ultrasound system. The ultrasound system 2 is any system that is able to perform imaging of part of a body of a subject using ultrasound. The ultrasound system 2 comprises an ultrasound probe that is to be placed in contact with the part of the body of the subject to be imaged, and that emits ultrasound and records the reflections. The ultrasound system 2 processes one or more signals representing the reflections to generate a series of ultrasound images of the part of the body of the subject.

A patient monitoring system 4 is provided that obtains measurements of one or more physiological characteristics of the subject. As shown in Fig. 1, the patient monitoring system 4 comprises a patient monitor 6, a biosensor 8 and a monitor central 10. In alternative embodiments, the patient monitoring system 4 can correspond to the patient monitor 6. For instance, the biosensor 8 is connected to the patient monitor 6. The biosensor 8 is configured to measure one or more physiological characteristics of the subject, and provides one or more signals representing the measurements of the physiological characteristic(s) to the patient monitor 6 for processing and/or display on a display screen of the patient monitor 6. The patient monitor 6 may be in the form of a bedside device (i.e. a device that is used at a patient's bedside in a hospital or other healthcare environment). In some embodiments, the biosensor 8 is integrated with the patient monitor 6, and is not a separate component.

The patient monitor 6 can be a device that acts as a data acquisition interface and a user interface that enables data interaction (with the physiological characteristic measurements) and viewing (of the measurements). The patient monitor 6 may be temporarily assigned to a patient or subject.

The physiological characteristic measured by the biosensor 8 can be any characteristic indicative of the health status of the subject, such as heart rate, blood pressure, blood oxygenation level, an ECG signal, an occurrence of an R-wave in an ECG signal, and occurrence of a T-wave in an ECG signal. In at least the latter three cases, the patient monitor 6 and biosensor 8 operate as, or are, an ECG machine/monitor.

The monitor central 10 is connected to the patient monitor 6 and receives physiological characteristic measurements from the patient monitor 6, as indicated by arrow 12. These measurements can be transmitted from the patient monitor 6 to the monitor central 10 continuously or periodically. As shown by arrow 14, the monitor central 10 can output the physiological characteristic measurement(s) to the ultrasound system 2. The monitor central 10 can also output (shown by arrow 16) the physiological characteristic measurement(s) and/or the signal(s) representing the physiological characteristics to a patient data storage system 18 that stores the measurements and/or signals in a patient record for the subject. In some embodiments, the monitor central 10 can compile the measurements and/or signals into a suitable file format, e.g. a Digital Imaging and Communications in Medicine (DICOM) format, and the file comprising the measurements and/or signals is sent to the patient data storage system 18.

In some embodiments, the monitor central 10 may be connected to multiple patient monitors that are monitoring different subjects, and therefore the monitor central 10 can act as a hub for the patient monitors 6. Thus, the monitor central 10 can be a central server that networks and manages a plurality of patient monitors 6 within a healthcare facility, and serves as a short-term repository for patient monitor vital signs data, and facilitates the transfer of this data to electronic medical records in the patient data storage system 18. The monitor central 10 can communicate with all patient monitors 6 within a healthcare facility, integrating data, and facilitating admit, discharge, and transfer events.

In some alternative embodiments to those shown in Fig. 1, the monitor central 10 may be omitted, and the functions performed thereby can be performed by the patient monitors 6 instead. Thus, the patient monitor 6 may send the physiological characteristic measurement(s) and/or the signal(s) representing the physiological characteristics directly to the patient data storage system 18. In these embodiments, the patient monitoring system 4 may just comprise a single patient monitor 6 (with a separate or integrated sensor 8).

To facilitate the use of the physiological characteristic measurements by the ultrasound system 2, it is preferable for an internal clock of the ultrasound system 2 and an internal clock of the patient monitoring system 4 to be synchronized by a time server 20. The time server 20 may be Internet-based (i.e. accessible via the Internet), or may be a part of the healthcare facilities internal network. In the latter case, the time server 20 may itself access an external Internet-based time server. The time server 20 may operate according to a Network Time Protocol (NTP). Thus, the ultrasound system 2 and patient monitoring system 4 are able to perform synchronization operations with the time server 20 to synchronize their internal clocks. A time server 20 (e.g. an NTP server) is a computer or server that supports a network time protocol and hosts a reference clock to which any device within the network can synchronize. The time server 20 can enable systems with variable latency networks to achieve millisecond-level synchronization. Typically, devices poll time servers at minimum polling times of roughly 64 seconds. According to the "xntpd" implementation of the NTP protocol, each polling time "disciplines" the clock of the system using a combination of direct modification, gradual slewing, offset adjustment, or hardpps interrupt service modification.

In some embodiments, the patient monitor(s) 6 and the monitor central 10 can perform respective synchronization operations with the time server 20. In alternative embodiments, the monitor central 10 can perform a synchronization operation with the time server 20, and communicate with patient monitors 6 any information needed to perform respective time synchronization operations, and/or any information needed to synchronize the patient monitors 6 so that the patient monitors 6 do not need to implement a network time protocol themselves.

In accordance with embodiments of the techniques described herein, the ultrasound system 2 is configured to query the patient monitoring system 4 (e.g. the monitor central 10) for vital signs data corresponding to a specific subject over a specific time interval. For the ultrasound system 2 to receive the physiological characteristic measurement(s) in real time or near real time, the connection 14 needs to be a low latency wireless connection.

As shown by signal 22, the ultrasound system 2 can send the ultrasound images to the patient data storage system 18. In some embodiments, the ultrasound system 2 can compile the ultrasound images into a suitable file format, e.g. a DICOM format, and the file comprising the images is sent to the patient data storage system 18. In some embodiments, the file compiled by the ultrasound system 2 can also include the physiological characteristic measurement(s) received from the patient monitoring system 4.

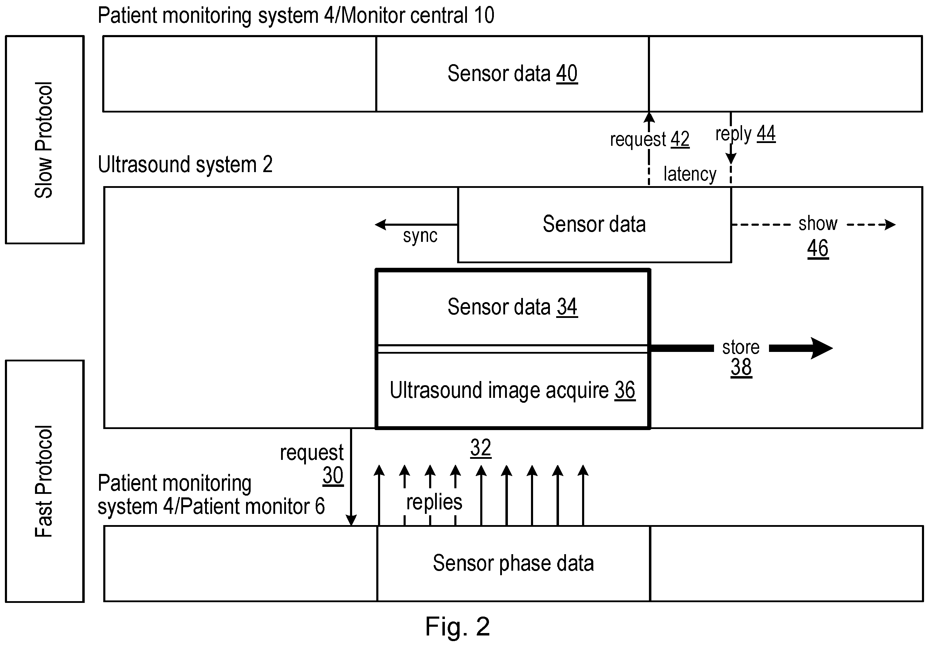

The diagram in Fig. 2 illustrates data exchange between the ultrasound system 2 and the patient monitoring system 4 when providing physiological characteristic measurements from the patient monitoring system 4 to the ultrasound system 2 to enable ultrasound images and synchronized physiological characteristic measurements to be displayed and/or stored in a data file for the patient. Fig. 2 shows two different communication protocols according to the techniques described herein, a "fast protocol" and a "slow protocol". The fast protocol can be used if the ultrasound system 2 is to use derived data from the patient monitor 6 with very low latency, for example, in order to guide ultrasound image acquisition actions. The slow protocol can be used when the ultrasound system 2 is to use data from the patient monitor 6 for real time display and file (e.g. DICOM) capture integration of vital signs.

In Fig. 2 the actions of the ultrasound system 2 in both protocols are shown in the middle portion, the actions of the patient monitoring system 4 in the slow protocol are shown in the top portion, and the actions of the patient monitoring system 4 in the fast protocol are shown in the bottom portion. In the slow protocol, the patient monitor 6 or monitor central 10 parts of the patient monitoring system 4 can communicate with the ultrasound system 2, and in the fast protocol, the patient monitor 6 can communicate with the ultrasound system 2.

In the fast protocol, the ultrasound system 2 is to use data derived from the patient monitor 6 with very low latency in order to, for example, guide acquisition actions. For example, ultrasound systems currently need QRST ECG phase information to trigger contrast and 3D imaging acquisition protocols. This can be achieved using the fast protocol described herein. The ultrasound system 2, and optionally also the patient monitor 6 and/or monitor central 10, perform a time synchronization operation to the time server to update their internal time clocks. This synchronization can be performed before the method below commences, or as one of the initial steps. The steps involved in this fast protocol are as follows:
The user of the ultrasound system 2 enters information for the subject to be scanned. The information for the subject can be an identifier for the subject, such as a medical record number (MRN), or other unique patient identifier.

The ultrasound system 2 discovers a patient monitor 6 that is actively monitoring that subject (e.g. via matching the subject's unique patient identifier with the identifier of the patient using or associated with the patient monitor 6. This identification step can be performed using a wireless communication protocol, such as Bluetooth, as indicated by signal 24 in Fig 1. This identification step can be initiated by the ultrasound system 2. In some embodiments, this identification step can be performed via a low latency wireless communication protocol, such as Bluetooth LE LC3. Once the appropriate patient monitor 6 has been identified, the ultrasound system 2 and the patient monitor 6 establish a wireless communication link. The wireless communication link is such that information or data is transmitted via the link using a digital communication protocol in an application layer of a protocol stack for the wireless communication link (i.e. an application layer in the 7-layer Open Systems Interconnection (OSI) network model). The digital communication protocol can be, for example, the Digital Navigation Link protocol, an information stream that pairs meta-data with samples.

The ultrasound system 2 requests measurements from the patient monitor 6 for a set of physiological characteristics for the subject. The request for measurements from the patient monitor 6 is shown by signal 30 in Fig. 2. The physiological characteristics can be any of heart rate, blood pressure, blood oxygenation level, an ECG signal, or ECG-signal specific characteristics, such as occurrence of an R-wave and/or T-wave. The fast protocol is particularly useful for rapidly changing or occurring physiological characteristics, such as the waves in the ECG signal. The request 30 typically requests the patient monitor 6 to continuously send (ping) measurements to the ultrasound system 2.

The patient monitor 6 responds to the request 30 with confirmation that the requested measurements are/will be available. This response is not shown in Fig. 2.

The patient monitor 6 begins streaming the requested data (physiological characteristic measurements) to the ultrasound system 2, with the lowest possible latency provided for by the wireless communication protocol. The streaming of the physiological characteristic measurements is shown by signals 32. The aim is for the data stream to arrive at the ultrasound system 2 in a timely manner from the patient monitor 6 so that the data/samples can be used, and so a latency of less than 50 ms would suffice. An ultrasound system 2 normally operates with some latency from acquisition of the ultrasound images to the display of those images, so the ultrasound system 2 can tolerate sample packetization latencies and communication latencies to some degree.

The ultrasound system 2 receives the physiological characteristic measurements (shown as block 34). In some embodiments, the ultrasound system 2 uses the received physiological characteristic measurements to govern ultrasound image acquisition behavior (e.g. R-wave gated imaging).

The ultrasound system 2 acquires ultrasound images 36 either as the physiological characteristic measurements 34 are being received from the patient monitor 6 in step 6, or, as noted, in response to certain received physiological characteristic measurements (i.e. occurrence of an R-peak or R-wave).

The ultrasound system 2 terminates the connection when no longer needed, for example when the ultrasound imaging process is stopped or otherwise complete.

The ultrasound system 2 sends the received physiological characteristic measurements and ultrasound images to the patient data storage system 18 for storage, as shown by signal 38. In some embodiments, the ultrasound system 2 compiles the physiological characteristic measurements and the ultrasound images into a digital file, for example in the DICOM format, and sends the digital file to the patient data storage system 18.

In the slow protocol, the ultrasound system 2 is to use data from the patient monitor 6 for real time display and/or capture of physiological characteristic measurements for integration into a single digital file (e.g. a file in the DICOM format). For example, users of an ultrasound system 2 may desire to have a heart rate displayed to provide clinical context for the examination, and for that heart rate data to be embedded in the saved file (DICOM). The ultrasound system 2, and optionally also the patient monitor 6 and/or monitor central 10, perform a time synchronization operation to the time server to update their internal time clocks. This synchronization can be performed before the method below commences, or as one of the initial steps. The steps involved in this slow protocol are as follows:
The user of the ultrasound system 2 enters information for the subject to be scanned. The information for the subject can be an identifier for the subject, such as a MRN, or other unique patient identifier.

The ultrasound system 2 initiates a connection to the monitor central 10 via a wired or wireless communication link to transfer patient information for a patient for the given MRN.

The ultrasound system 2 commences ultrasound imaging of the subject, and the patient monitor 6 obtains measurements of one or more physiological characteristics. The measurements obtained by the patient monitor 6 (and optionally sent to the monitor central 10) are shown as sensor data 40 in Fig. 2.

The ultrasound system 2 requests (signal 42) physiological characteristic measurements for the subject being imaged via the wireless communication link. Where the ultrasound system 2 sends the request to the monitor central 10, the ultrasound system 2 includes the information identifying the subject (e.g. MRN) in the request 42. In some embodiments, the request 42 requests physiological characteristic measurements (also referred to as a "wave snippet") relating to a defined time interval, for example 100 milliseconds ago until now (i.e. the time at which the request 42 is sent).

The monitor central 10 sends the requested physiological characteristic measurements (i.e. the requested wave snippet) to the ultrasound system 2, as shown by reply 44. The requested physiological characteristic measurements can comprise one or more timestamps indicating the time(s) of acquisition of the measurements by the patient monitor 6. The timestamps may comprise numeric and/or Coordinated Universal Time (UTC) timestamps.

Using the timestamps, the ultrasound system 2 synchronizes the received physiological characteristic measurements to the acquired ultrasound images, and displays 46 the physiological characteristic measurements (e.g. a received wave snippet) and the ultrasound images on the screen of the ultrasound system 2.

When the ultrasound system 2 stops imaging (e.g. the scanning of the subject is complete) a capture event is triggered, and the ultrasound images and physiological characteristic measurements are compiled into a file for sending to the patient data storage system 18. The file may be a DICOM file. The physiological characteristic measurements (e.g. wave snippet) can be embedded into the DICOM metadata with the associated timestamps (e.g. UTC timestamps) that are time aligned with the ultrasound images.

In some embodiments, the slow and fast protocols (i.e. synchronized data exchange) can trigger anatomic ultrasound scanning in response to a change of a vital sign, e.g. a change in heart rate. This trigger can have a latency of less than, for example, 100 milliseconds, although other latencies (higher or lower) can be used.

In some embodiments, the ultrasound system 2 and patient monitoring system 4 can implement both the fast protocol and the slow protocol at the same time.

In some embodiments, a review mode is provided. In the review mode:
A user interacts with an ultrasound image review station, which can be associated with the patient data storage system 18.

The user selects a clinical application package that requires physiological characteristic measurements (e.g. wave data) that was not included in a primary DICOM capture (i.e. a capture of the data representing the original acquisition).

The review station initiates a connection to the patient monitoring system 4 (e.g. the monitor central 10) and requests information for the subject of interest using information identifying the subject, e.g. using a MRN.

The review station sends a request for physiological characteristic measurements (e.g. a wave snippet) similar to that implemented in the slow protocol.

The user at the review station completes its analysis, and generates a DICOM Secondary Capture file which encapsulates the quantification results (i.e. a capture of data derived from a primary capture or some other data source).

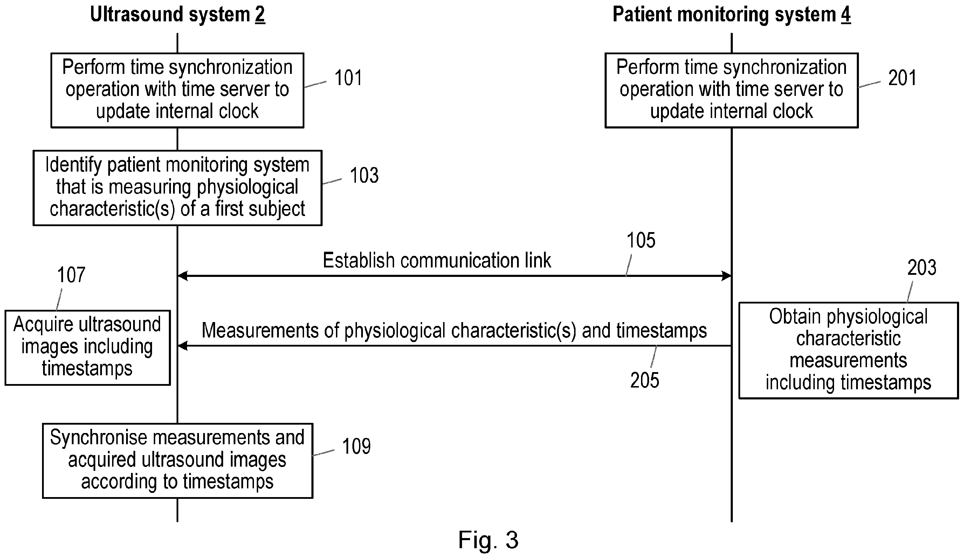

Fig. 3 is a signaling diagram illustrating the operations of an ultrasound system 2 and a patient monitoring system 4 according to various embodiments. The ultrasound system 2 is for acquiring ultrasound images of a first subject and the patient monitoring system 4 is for obtaining measurements of one or more physiological characteristics of the first subject. The patient monitoring system 4 may be configured to perform ECG measurements. In some embodiments, the one or more physiological characteristics measured by the patient monitoring system 4 comprise one or more haemodynamic characteristics, such as: heart rate, blood pressure, blood oxygenation level, occurrence of an R-wave, and occurrence of a T-wave.

In step 101, the ultrasound system 2 performs a time synchronization operation with an Internet-based time server to update an internal clock of the ultrasound system 2. Similarly, in step 201, the patient monitoring system 4 performs a time synchronization operation with an Internet-based time server to update an internal clock of the patient monitoring system 4.

In step 103, the ultrasound system 2 identifies a patient monitoring system (and in particular a patient monitor 6) that is measuring one or more physiological characteristics of the first subject. Step 103 results in the ultrasound system 2 identifying patient monitoring system 4. In some embodiments, step 103 comprises the ultrasound system 2 receiving an identifier for the first subject (for example by the identifier being input to the ultrasound system 2 by an operator) and the ultrasound system 2 queries a plurality of patient monitoring systems to identify a patient monitoring system that is measuring one or more physiological characteristics of a subject having the identifier of the first subject. In some embodiments, the identifier is a MRN.

The ultrasound system 2 and the patient monitoring system 4 establish a communication link with each other (step 105). In some embodiments, the communication link is a wireless communication link. In these embodiments, the measurements of the physiological characteristics are conveyed to the ultrasound system 2 using a digital communication protocol in an application layer of a protocol stack for the wireless communication link. In these embodiments, the digital communication protocol can convey the measurements in a digital format to the ultrasound system 2. In some embodiments, the wireless communication link uses Bluetooth or Wi-Fi.

The patient monitoring system 4 obtains a series of measurements of the one or more physiological characteristics of the first subject (step 203). The measurements comprise respective timestamps indicating respective times of acquisition of the measurements by the patient monitoring system 4.

The patient monitoring system 4 sends the series of timestamped measurements of the one or more physiological characteristics of the first subject to the ultrasound system 2 (step 205). In some embodiments, the timestamped measurements are sent to the ultrasound system 2 as they are acquired, e.g. in real-time.

At the ultrasound system 2, the series of measurements of the one or more physiological characteristics of the first subject are received from the patient monitoring system 4.

The ultrasound system 2 acquires a series of ultrasound images of the first subject (step 107). The ultrasound images comprise respective timestamps, determined according to the internal clock, indicating respective times of acquisition of the ultrasound images. In some embodiments, step 107 is performed while the series of measurements are received from the patient monitoring system 4. In alternative embodiments, the ultrasound system 2 can acquire individual ultrasound images in response to specific measurements in the received series of measurements. For example, the received series of measurements can indicate when an R-wave occurs, and the occurrence of the R-wave can trigger the ultrasound system 2 to obtain one or more ultrasound images.

In step 109, the ultrasound system 2 then synchronizes the received series of measurements with the acquired series of ultrasound images according to the respective timestamps.

In some embodiments, after synchronizing the measurements and ultrasound images in step 109, the ultrasound system 2 can display the synchronized series of ultrasound images and the series of measurements on a display unit of the ultrasound system 2.

In some embodiments, the ultrasound system 2 can embed the synchronized images and measurements into a data file and send the data file to a patient data storage system 18. The patient data storage system 18 stores the data file with other information and/or data files for the first subject. In some embodiments the data file is a DICOM file.

In some embodiments, after the ultrasound system 2 has identified the patient monitoring system 4 in step 103, the ultrasound system 2 can send a query to the patient monitoring system 4 comprising the identifier for the first subject and requesting a communication link with a patient monitoring system 4. The patient monitoring system 4 can send a response to the ultrasound system 2 to initiate establishment of the communication link in step 105.

In some embodiments, the patient monitoring system 4 sends the series of measurements in step 205 in response to receiving a request from the ultrasound system 2 for recently-obtained measurements of the physiological characteristic(s) of the first subject.

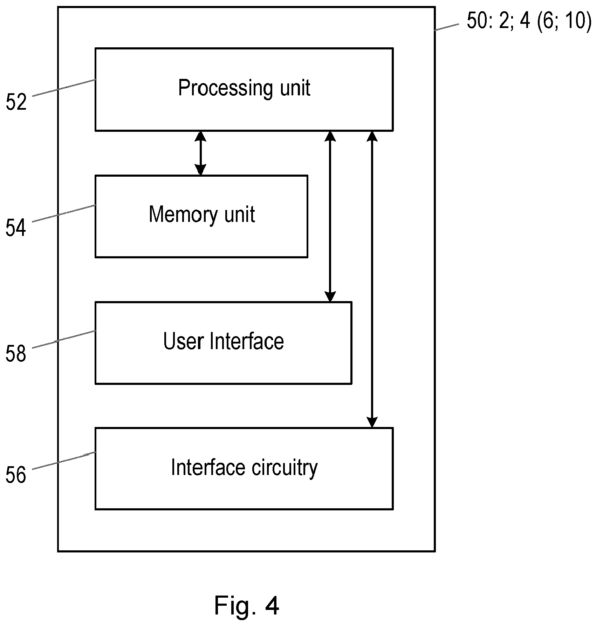

Fig. 4 is a simplified block diagram illustrating components of an apparatus 50 according to various embodiments. Any of the ultrasound system 2, patient monitoring system 4, patient monitor 6 in a patient monitoring system 4, and monitor central 10 in a patient monitoring system 4 can be implemented as shown by the apparatus 50 in Fig 4.

The apparatus 50 includes a processing unit 52 that controls the operation of the apparatus 50 and that can be configured to execute or perform the methods described herein. The processing unit 52 can be implemented in numerous ways, with software and/or hardware, to perform the various functions described herein. For example, the processing unit 52 can implement the functions of any of the ultrasound system 2, patient monitoring system 4, patient monitor 6 or monitor central 10 described above with respect to Figs. 1-3. The processing unit 52 may comprise one or more microprocessors or digital signal processors (DSPs) that may be programmed using software or computer program code to perform the required functions and/or to control components of the processing unit 52 to effect the required functions. The processing unit 52 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 52 is connected to a memory unit 54 that can store data, information and/or signals for use by the processing unit 52 in controlling the operation of the apparatus 50 and/or in executing or performing the methods described herein. Thus, the memory unit 54 can store measurements of physiological characteristic(s) and/or ultrasound images. In some implementations the memory unit 54 stores computer-readable code that can be executed by the processing unit 52 so that the processing unit 52 performs one or more functions, including the methods described herein. The memory unit 54 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 54 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 50 also includes interface circuitry 56 for enabling a wireless communication link to be established with other apparatus(es) 50 and for data to be exchanged with those other apparatus(es) 50 as described herein. In the case of a wireless connection, the interface circuitry 56 (and thus apparatus 50) may include one or more suitable antennas for transmitting/receiving over a transmission medium (e.g. the air). The interface circuitry 56 is connected to the processing unit 52 to enable information or data received by the interface circuitry 56 to be provided to the processing unit 52, and/or information or data from the processing unit 52 to be transmitted by the interface circuitry 56.

In some embodiments, the apparatus 50 comprises a user interface 58 that includes one or more components that enables a user of apparatus 50 to input information, data and/or commands into the apparatus 50 (for example a command to start acquisition of ultrasound images or physiological characteristic measurements, or information such as an identifier of the subject of interest), and/or enables the apparatus 50 to output information or data to the user of the apparatus 50 (for example by displaying the ultrasound images, physiological characteristic measurements or a (synchronized) combination thereof. The user interface 58 can comprise any suitable input component(s), including but not limited to a keyboard, keypad, one or more buttons, switches or dials, a mouse, a track pad, a touchscreen, a stylus, a camera, a microphone, etc., and the user interface 58 can comprise any suitable output component(s), including but not limited to a display screen, one or more lights or light elements, one or more loudspeakers, a vibrating element, etc.

It will be appreciated that a practical implementation of any of an ultrasound system 2, patient monitoring system 4, patient monitor 6 or monitor central 10 will include additional components to those shown in Fig. 4.

Therefore there is provided methods for providing physiological characteristic measurements from a patient monitoring system to an ultrasound system to enable ultrasound images and synchronized physiological characteristic measurements to be displayed and/or stored in a data file for the patient. Also provided are an ultrasound system and patient monitoring system for implementing these methods.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of operating an ultrasound system (2) that is for acquiring ultrasound images of a first subject, the method comprising:
performing (101) a time synchronization operation with an Internet-based time server (20) to update an internal clock of the ultrasound system (2);
identifying (103) a patient monitoring system (4; 6) that is measuring one or more physiological characteristics of the first subject;
establishing (105) a communication link with the identified patient monitoring system (4; 6);
receiving (205) a series of measurements of the one or more physiological characteristics of the first subject from the patient monitoring system (4; 6), wherein the received series of measurements comprise respective timestamps indicating respective times of acquisition of the measurements by the patient monitoring system (4; 6);
acquiring (107) a series of ultrasound images of the first subject, wherein the ultrasound images comprise respective timestamps, determined according to the internal clock, indicating respective times of acquisition of the ultrasound images; and
synchronizing (109) the received series of measurements with the acquired series of ultrasound images according to the respective timestamps.

2. A method as claimed in claim 1, wherein the communication link is a wireless communication link and the measurements are conveyed to the ultrasound system (2) using a digital communication protocol in an application layer of a protocol stack for the wireless communication link.

3. A method as claimed in claim 2, wherein the digital communication protocol conveys the measurements in a digital format to the ultrasound system (2).

4. A method as claimed in any of claims 1-3, wherein the method further comprises: displaying, via a display unit (56), the synchronized series of ultrasound images and the series of measurements.

5. A method as claimed in any of claims 1-4, wherein the method further comprises:
embedding the synchronized images and measurements into a data file; and
sending the data file to a patient data storage system (18).

6. A method as claimed in any of claims 1-5, wherein the step of identifying (103) a patient monitoring system (4; 6) comprises receiving an identifier for the first subject and querying a plurality of patient monitoring systems (4; 6) to identify a patient monitoring system (4; 6) that is measuring one or more physiological characteristics of a subject having the identifier of the first subject.

7. A method as claimed in any of claims 1-6, wherein the step of acquiring (107) comprises acquiring individual ultrasound images in response to specific measurements in the received series of measurements.

8. A method of operating a patient monitoring system (4; 6) that is for obtaining measurements of one or more physiological characteristics of a first subject, the method comprising:
performing (201) a time synchronization operation with an Internet-based time server (20) to update an internal clock of the patient monitoring system (4; 6);
establishing (105) a communication link with an ultrasound system (2) that is to acquire ultrasound images of the first subject;
obtaining (203) a series of measurements of the one or more physiological characteristics of the first subject, wherein the measurements comprise respective timestamps indicating respective times of acquisition of the measurements by the patient monitoring system (4; 6); and
sending (205) the series of timestamped measurements of the one or more physiological characteristics of the first subject to the ultrasound system (2).

9. A method as claimed in claim 8, wherein the communication link is a wireless communication link and the measurements are conveyed to the ultrasound system (2) using a digital communication protocol in an application layer of a protocol stack for the wireless communication link.

10. A method as claimed in claim 8 or 9, wherein the method further comprises:
receiving a query from the ultrasound system (2), the query comprising an identifier for the first subject and requesting a communication link with a patient monitoring system (4; 6) that is measuring one or more physiological characteristics of the first subject; and
sending a response to the ultrasound system (2) to initiate establishment of the communication link.

11. A method as claimed in any of claims 8-10, wherein the series of timestamped measurements of the one or more physiological characteristics of the first subject are sent to the ultrasound system (2) as they are acquired.

12. A method as claimed in any of claims 8-11, wherein the method further comprises:
receiving, from the ultrasound system (2), a request for recently-obtained measurements of the one or more physiological characteristics of the first subject; and
wherein the step of sending is performed in response to receiving the request.

13. A computer program product comprising a computer readable medium having computer readable code embodied therein, the computer readable code being configured such that, on execution by a suitable computer or processor, the computer or processor is caused to perform the method of any of claims 1-12.

14. An ultrasound system (2) that is for acquiring ultrasound images of a first subject, the ultrasound system (2) configured to:
perform a time synchronization operation with an Internet-based time server (20) to update an internal clock of the ultrasound system (2);
identify a patient monitoring system (4; 6) that is measuring one or more physiological characteristics of the first subject;
establish a communication link with the identified patient monitoring system (4; 6);
receive a series of measurements of the one or more physiological characteristics of the first subject from the patient monitoring system (4; 6), wherein the received series of measurements comprise respective timestamps indicating respective times of acquisition of the measurements by the patient monitoring system (4; 6);
acquire a series of ultrasound images of the first subject, wherein the ultrasound images comprise respective timestamps, determined according to the internal clock, indicating respective times of acquisition of the ultrasound images; and
synchronize the received series of measurements with the acquired series of ultrasound images according to the respective timestamps.

15. A patient monitoring system (4; 6) that is for obtaining measurements of one or more physiological characteristics of a first subject, the patient monitoring system (4; 6) configured to:
perform a time synchronization operation with an Internet-based time server (20) to update an internal clock of the patient monitoring system (4; 6);
establish a communication link with an ultrasound system (2) that is to acquire ultrasound images of the first subject;
obtain a series of measurements of the one or more physiological characteristics of the first subject, wherein the measurements comprise respective timestamps indicating respective times of acquisition of the measurements by the patient monitoring system (4; 6); and
send the series of timestamped measurements of the one or more physiological characteristics of the first subject to the ultrasound system (2).
